(19) Europäisches Patentamt
European Patent Office
Office européen des brevets

(11) Veröffentlichungsnummer : **0 231 367 B1**

# (12) EUROPÄISCHE PATENTSCHRIFT

(45) Veröffentlichungstag der Patentschrift : **24.07.91 Patentblatt 91/30**

(51) Int. Cl.$^5$ : **A61K 31/335**

(21) Anmeldenummer : **86905250.6**

(22) Anmeldetag : **29.07.86**

(86) Internationale Anmeldenummer : **PCT/EP86/00450**

(87) Internationale Veröffentlichungsnummer : **WO 87/00751 12.02.87 Gazette 87/04**

## (54) VERWENDUNG VON OXIRANCARBONSÄUREN ZUR BEHANDLUNG DER HYPERLIPÄMIE.

(30) Priorität : **02.08.85 DE 3527800**

(43) Veröffentlichungstag der Anmeldung : **12.08.87 Patentblatt 87/33**

(45) Bekanntmachung des Hinweises auf die Patenterteilung : **24.07.91 Patentblatt 91/30**

(84) Benannte Vertragsstaaten : **AT BE CH DE FR GB IT LI LU NL SE**

(56) Entgegenhaltungen :
**EP-A- 0 025 192**
**EP-A- 0 046 590**
**EP-A- 0 046 961**
**EP-A- 0 071 175**
**US-A- 4 324 796**

(56) Entgegenhaltungen :
**Biochemical Pharmacology, vol. 33, no. 3, 1984, (Pergamon Press Ltd,GB) P.P. Koundakjian et al.: "Metabolic changes in fed rats caused by chronic administration of ethyl 2(5'4-chlorophenyl' pentyl)oxirane-2-carboxylate, a new hypoglycaemic compound, pages 465-473, see page 465 cited in the application.**
**Diabetologia, vol. 29, no. 8, August 1986 H. Bliesath et al.: "First administration of ETOMOXIR in man", page 519 A see abstract no. 49**
**Biochemical Society Transactions, vol. 14, no. 4 August 1986, Y.T. Kruszynska et al.: "The effects of 2 ( -6(4-chlorophenoxy) hexyl) oxirane-2-carboxylate (extomir) on glucose turnover in fasted rats", pages 699-700 see the whole article**

(73) Patentinhaber : **Byk Gulden Lomberg Chemische Fabrik GmbH**
**Byk-Gulden-Strasse 2**
**W-7750 Konstanz (DE)**

(72) Erfinder : **WOLF, Horst**
**Im Tal 17**
**W-7753 Allensbach 4 (DE)**
Erfinder : **EISTETTER, Klaus**
**Säntisblick 7**
**W-7750 Konstanz 19 (DE)**

**EP 0 231 367 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Arzneimittel zur Behandlung der Hyperlipämie

Die Enfindung betrifft die neue Verwendung von bekannten Oxirancarbonsäuren zur Herstellung von lipidsenkenden Arzneimitteln. Die Arzneimittel werden zur Verhütung und Behandlung von Krankheiten eingesetzt, die auf einen erhöhten Cholesterin- und/oder Triglycerid-Konzentration beruhen.

Stand der Technik

In der EP-A-0 046 590 werden hypoglycämisch un hypoketonämisch wirksame Phen(alk)oxy-substituierte Oxirancarbonsäuren beschrieben, die zur Behandlung des Diabetes eingesetzt werden sollen. - In der Zeitschrift Biochemical Pharmacology 33, 465 (1984) wird über die cholesterin- und triglycerid-konzentrationssenkende Eigenschaft des 2-[5-(4-Chlorphenyl)-pentyl]-oxiran-2-carbonsäureethylesters berichtet.

Beschreibung der Erfindung

Es wurde nun gefunden, daß die aus der EP-A-0 046 590 bekannten Oxirancarbonsäuren eine deutliche Senkung der Cholesterin- und Triglycerid-Konzentration bewirken. Oberraschenderweise ist das Ausmaß der Senkung der Cholesterin- und Triglycerid-Konzentration wesentlich größer, als man dies aufgrund der für den 2-[5-(4-Chlorphenyl)-pentyl]-oxiran-2-carbonsäure-ethylester erhobenen Daten hätte erwarten können. Die überraschend starke Senkung der Cholesterin- und Triglycerid-Konzentration läßt die aus der EP-A-0 046 590 bekannten Oxirancarbonsäuren für den Einsatz bei der Verhütung und Behandlung von solchen Krankheiten geeignet erscheinen, die auf einer erhöhten Cholesterin- und/oder Triglycerid-Konzentration beruhen.

Gegenstand den Erfindung ist daher die Verwendung von Oxirancarbonsäuren den Formel I

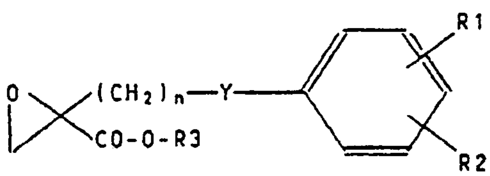 (I),

worin

R1 ein Wasserstoffatom, ein Halongenatom, eine 1-4C-Alkylgruppe, eine 1-4C-Alkoxygruppe, eine Nitrogruppe oder eine Trifluormethylgruppe bedeudet,

R2 eine der Beudeutungen von R1 hat,

R3 ein Wasserstoffatom oder eine 1-4C-Alkylgruppe,

Y die Gruppierung $-O-(CH_2)_{m}-$,

m 0 oder eine ganze Zahl von 1 bis 4 und

n eine ganze Zahl von 2 bis 8 bedeuten,

wobei die Summe von m und n eine ganze Zahl von 2 bis 8 ist, und den pharmakologisch verträglischen Salzen der Carbonsäuren, zur Herstellung von Arzneimitteln für die Verhütung und/oder Behandlung von Krankheiten, die auf einer erhöhten Cholesterin- und/oder Triglycerid-Konzentration beruhen.

Als 1-4C-Alkylgruppen kommen geradkettige oder verzweigte Alkylreste mit 1 bis 4 Kohlenstoffatomen in Betracht. Geradkettige Alkylreste sind beispielsweise der Methyl-, Ethyl-, n-Propyl- und n-Butylrest, von denen der Methyl- und der Ethylrest bevorzugt sind. Verzweigte Alkylreste sind beispielsweise der Isopropyl-, Isobutyl-, sek. -Butyl- und tert. -Butylrest. Als Alkylreste von 1-4C-Alkoxygruppen kommen sowohl geradkettige als auch verzweigte Niederalkylgruppen in Frage. Die Methoxygruppe ist als 1-4C-Alkoxygruppe bevorzugt.

Halogenatome sind Fluor-, Chlor- und Bromatome, von denen Fluor, insbesondere Chlor bevorzugt sind.

Die Substituenten R1 und R2 des Phenylrings befinden sich bevorzugt in meta- oder para-Stellung zum (Alk)oxyalkylenoxirancarbonsäurerest.

Als Salze kommen Salze mit anorganischen und organischen Basen in Betracht. Als Kationen für die Salzbildung werden vor allem die Kationen der Alkamimetalle, Erdalkalimetalle oder Erdmetalle verwendet. Beispielsweise seien die Salze von Lithium, Natrium, Kalium, Magnesium, Calcium und Aluminium genannt.

Als Krankheiten, die auf einer erhöhten Cholesterin- und/oder Triglycerid- Konzentration (Hyperlipämie) beruhen, seien in erster Linie alle Formen der Arteriosklerose genannt, insbesondere Koronarsklerose und alle damit zusammenhängenden krankhaften Veränderungen, die unter dem Sammelbegriff "koronare Herzkrankheiten" zusammengefaßt werden können.

Bei der erfindungsgemäßen Verwendung der Verbindungen der Formel I zur Herstellung der vorstehend genannten Arzneimittel werden die Verbindungen der Formel I (=Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 95% beträgt.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten- Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z. B. Cyclodextrine) verwendet werden.

Die Wirkstoffe können oral oder parenterat appliziert werden.

Im allgemeinen hat es sich in der Humanmedizin als vorteilhaft erwiesen, den oder die Wirkstoffe bei oraler Gabe in einer Tagesdosis von etwa 0,1 bis etwa 30, vorsugsweise 0,3 bis 15, insbesondere 0,6 bis 3 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die Wirkstoffe zur Behandlung der oben genannten Krankheiten eingesetzt werden, so können die durch die erfindungsgemäße Verwendung der Wirkstoffe erhaltenen Arzneimittel auch einen oder mehrere andere pharmakologisch aktive Verbindungen, insbesondere andere sogenannte lipidsenkende Stoffe wie p-Aminosalicylsäure, D-Thyroxin, Nicotinsäure, Sitosterine, Colestyramin, Clofibrat, Ciprofibrat, Probucol, Colestipol, Gemfibrozil, Fenofibrat oder Bezafibrat, enthalten.

Eine Ausgestaltung der Erfindung ist die Verwendung von Oxirancarbonsäuren der Formel I, worin R1 und R2 meta- oder para-ständig sind und R1 ein Wasserstoffatom, ein Chloratom, eine Methylgruppe, eine Methoxygruppe, eine Nitrogruppe oder eine Trifluormethylgruppe, R2 ein Wasserstoffatom oder ein Chloratom, R3 ein Wasserstoffatom oder eine 1-4C-Alkylgruppe, Y die Gruppierung $-O-(CH_2)_m-$, m 0 oder 1 und n eine ganze Zahl von 3 bis 7 bedeuten, wobei die Summe von m und n eine ganze Zahl von 3 bis 7 ist, und den pharmakologisch verträglichen Salzen der Carbonsäuren, zur Herstellung von Arzneimitteln für die Verhütung und/oder Behandlung von Krankheiten, die auf einer erhöhten Cholesterin- und/oder Triglycerid-Konzentration beruhen.

Eine weitere Ausgestaltung der Erfindung ist die Verwendung von Oxirancarbonsäuren der Formel I, worin R1 meta- oder pare-ständig ist und R1 ein Wasserstoffatom, ein Chloratom oder eine Trifluormethylgruppe, R2 ein Wasserstoffatom, R3 ein Wasserstoffatom, eine Methylgruppe oder eine Ethylgruppe, Y die Gruppierung -O-, n eine ganze Zahl von 4 bis 6 bedeuten, und den pharmakologisch verträglichen Salzen der Carbonsäuren, zur Herstellung von Arzneimitteln für die Verhütung und/oder Behandlung von Krankheiten, die auf einer erhöhten Cholesterin- und/oder Triglycerid-Konzentration beruhen.

Eine bevorzugte Ausgestaltung der Erfindung ist die Verwendung einer oder mehrerer Oxirancarbonsäuren ausgewählt aus der Gruppe bestehend aus 2-[4-(3-Chlorphenoxy)-butyl]-oxiran-2-carbonsäureethylester, 2-[4-(3-Trifluormethylphenoxy)-butyl]-oxiran-2-carbonsäureethylester, 2-[6-(4-Chlorphenoxy)-hexyl]-oxiran-2-carbonsäureethylester, 2-[5-(4-Chlorphenoxy)-pentyl]-oxiran-2-carbonsäureethylester, 2-[6-(3,4-Dichlorphenoxy)-hexyl]-oxiran-2-carbonsäureethylester, 2-[6-4-Fluorphenoxy)-hexyl]-oxiran-2-carbonsäureethylester und 2-(6-Phenoxyhexyl)-oxiran-2-carbonsäureethylester sowie den entsprechenden Oxiran-2-carbonsäuren und ihren pharmakologisch verträglichen Salzen, zur Herstellung von Arzneimitteln für die Verhütung und/oder Behandlung von Krankheiten, die auf einer erhöhten Cholesterin- und/oder Triglycerid-Konzentration beruhen.

Eine besonders bevorzugte Ausgestaltung der Erfindung ist die Verwendung von 2-[6-(4-Chlorphenoxy)hexyl]-oxiran-2-carbonsäureethylester zur Herstellung von Arzneimitteln für die Verhütung und/oder Behandlung von Krankheiten, die auf einer erhöhten Cholesterin- und/oder Triglycerid-Konzentration beruhen.

## Beispiel für eine pharmazeutische Formulierung

Weichgelatinekapseln mit einem Gehalt von 100mg

525 g 2-[6-(4-Chlorphenoxy)hexyl]-oxiran-2-carbonsäureethylester werden mit 1764 g einer Mischung aus Emulgator (z. B. Cremophor® RH40) und Neutralöl (z. B. Miglyol 812) - im Verhältnis von z. B. 1 :16 bis 16 :1 sorgfältig gemischt. Jeweils 436 mg der Mischung werden in Weichgelatinekapseln der Größe 8 abgefüllt.

## Pharmakologie

In ihren cholesterin- und triglycerid-konzentrationssenkenden Eigenschaften zeigen sich im Tierversuch die Verbindungen der Formel I (insbesondere der 2-[6-(4-Chlorphenoxy)hexyl]-oxiran-2-carbonsäureethylester = Verbindung B) dem 2-[5(4-Chlorphenyl)pentyl]-oxiran-2-carbonsäureethylester = Verbindung A in überraschender Weise deutlich überlegen. Diese Überlegenheit geht aus der Gegenüberstellung der Cholesterinsenkung im Serum nüchterner Ratten hervor (Tab. 1), die nach einmaliger oraler Applikation von 0.05 mmol/kg der Verbindungen zeitabhängig ermittelt wurde.

Tabelle 1

| Verbindung | % Veränderung des Plasmacholesterins im Vergleich zur Kontrollgruppe (n = 12) | | |
|---|---|---|---|
| | 2-3 | 4-5 | 24 h p. appl. |
| A | + 14 | + 12 | - 30 |
| B | + 6 | - 16 | - 46 |

Die Ermittlung der pharmakologischen Daten erfolgte nach folgenden Methoden :

1. Dosierung

Einmalige Gabe von 0.05 mmol/kg der Verbindung A in wäßriger Lösung bzw. B in wäßriger Emulsion unter Zusatz von 2 Gewichtsteilen Cremophor®-EL (BASF, Ludwigshafen). Das Applikationsvolumen betrug 10 ml/kg und wurde mittels Magensonde verabreicht.

2. Versuchstiere

Als Versuchstiere wurden männliche SD-Ratten der SPF-Zucht Ivanovas (Kisslegg) mit einer Körpermasse von 255-440 g verwendet. Die Haltung der Tiere erfolgte konventionell zu je 4 Tieren in Makrolonkäfigen (22 x 38 cm) in einem temperierten Raum (21-23°C) mit festem Tag/Nacht-Rhythmus (7.00/19.00 Uhr) und regulierter rel. Luftfeuchtigkeit von 55-60 %. Die Tiere erhielten bis 24 Stunden vor Versuchsbeginn Haltungsdiät Altromin 1324 der Fa. Altromin (Lage). Wasser erhielten die Tiere ad libitum.

5. Methodik

Die Tiere wurden randomisiert in Gruppen zu je 12 Tieren eingeteilt. Die Kontrollgruppe erhielt Wasser statt Substanzlösung. Der Versuch wurde morgens mit der Blutabnahme für die Vorwertbestimmung begonnen. Sofort anschließend erfolgte die Substanzgabe und zu den in Tab. 1 angegebenen Zeitpunkten erfolgten weitere Blutabnahmen retroorbital für die Cholesterinbestimmung.

4. Bestimmungsmethode

Die Bestimmung des Cholesterins erfolgte im Plasma, das durch Zentrifugation der heparinisierten Blutproben gewonnen wurde, nach der CHOD-PAP- Methode nach Trinder, P. : Ann. Clin. Biochem. 6, 24 (1969) mittels der Testkombination der Fa. Boehringer/Mannheim, Best. Nr. 187 313.

5. Auswertung

Die Auswertung erfolgte mittels eines Computerprogrammes ("Screeny"), das nach Elimination von Extremwerten nach dem Verfahren von Dixon, W.J. : Biometrics 9, 74 (1953) Mittelwerte, Standardabweichungen und prozentuale Veränderungen gegenüber der Kontrollgruppe berechnet. Letztere sind in Tab. 1 angegeben.

**Patentansprüche**

1. Verwendung von Oxirancarbonsäuren der Formel I

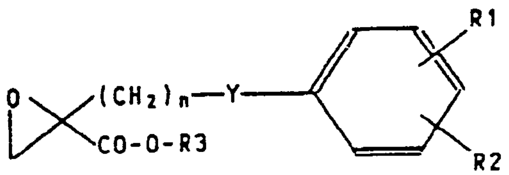

(I),

worin

R1 ein Wasserstoffatom, ein Halogenatom, eine 1-4C-Alkylgruppe, eine 1-4C-Alkoxygruppe, eine Nitrogruppe oder eine Trifluormethylgruppe bedeutet

R2 eine der Bedeutungen von R1 hat,

R3 ein Wasserstoffatom oder eine 1-4C-Alkylgruppe,

Y die Gruppierung $-O-(CH_2)_{m}-$,

m 0 oder eine ganze Zahl von 1 bis 4 und

n eine ganze Zahl von 2 bis 8 bedeuten,

wobei die Summe von m und n eine ganze Zahl von 2 bis 8 ist, und den pharmakologisch verträglichen Salzen der Carbonsäuren, zur Herstellung von Arzneimitteln für die Verhütung und/oder Behandlung von Krankheiten, die auf einer erhöhten Cholesterin- und/oder Triglycerid-Konzentration beruhen.

2. Verwendung von Oxirancarbonsäuren der Formel I, worin R1 und R2 metaoder para-ständig sind und R1 ein Wasserstoffatom, ein Chloratom, eine Methylgruppe, eine Methoxygruppe, eine Nitrogruppe oder eine Trifluormethylgruppe, R2 ein Wasserstoffatom oder ein Chloratom, R3 ein Wasserstoffatom oder eine 1-4C-Alkylgruppe, r die Gruppierung $-O-(CH_2)_{m}-$, m 0 oder 1 und n eine ganze Zahl von 3 bis 7 bedeuten, wobei die Summe von m und n eine ganze Zahl von 3 bis 7 ist, und den pharmakologisch verträglichen Salzen der Carbonsäuren, zur Herstellung von Arzneimitteln für die Verhütung und/oder Behandlung von Krankheiten, die auf einer erhöhten Cholesterin- und/oder Triglycerid-Konzentration beruhen.

3. Verwendung von Oxirancarbonsäuren der Formel I, worin R1 meta- oder para-ständig ist und R1 ein Wasserstoffatom, ein Chloratom oder eine Trifluormethylgruppe, R2 ein Wasserstoffatom, R3 ein Wasserstoffatom, eine Methylgruppe oder eine Ethylgruppe, r die Gruppierung -O-, n eine ganze Zahl von 4 bis 6 bedeuten, und den pharmakologisch verträglichen Salzen der Carbonsäuren, zur Herstellung von Arzneimitteln für die Verhütung und/oder Behandlung von Krankheiten, die auf einer erhöhten Cholesterin- und/oder Triglycerid-Konzentration beruhen.

4. Verwendung einer oder mehrerer Oxirancarbonsäuren ausgewählt aus der Gruppe bestehend aus 2-(4-(3-Chlorphenoxy)-butyl]-oxiran-2-carbonsäureethylester, 2-(4-(3-Trifluormethylphenoxy)-butyl]-oxiran-2-carbonsäureethylester, 2-(6-(4-Chlorphenoxy)-hexyl]-oxiran-2-carbonsäureethylester, 2-(5-(4-Chlorphenoxy)-pentyl]-oxiran-2-carbonsäureethylester, 2-(6-(3,4-Dithlorphenoxy)-hexyl]-oxiran-2-carbonsäureethylester, 2-[6-(4-Fluorphenoxy)-hexyl]-oxiran-2-carbonsäureethylester und 2-(6-Phenoxyhexyl)-oxiran-2-carbonsäureethylester sowie den entsprechenden Oxiran-2-carbonsäuren und ihren pharmakologisth verträglichen Salzen, zur Herstellung von Arzneimitteln für die Verhütung und/oder Behandlung von Krankheiten, die auf einer erhöhten Cholesterin- und/oder Triglycerid-Konzentration beruhen.

5. Verwendung von 2-(6-(4-Chlorphenoxy)hexyl]-oxiran-2-carbonsäureethylester zur Herstellung von Arzneimitteln für die Verhütung und/oder Behandlung von Krankheiten, die auf einer erhöhten Cholesterin- und/oder Triglycerid-Konzentration beruhen.

6. Verwendung von Oxirancarbonsäuren der Formel I

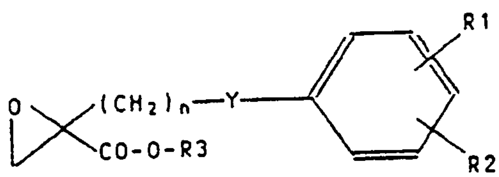

(I),

worin

R1 ein Wasserstoffatom, ein Halogenatom, eine 1-4C-Alkylgruppe, eine 1-4C-Alkoxygruppe, eine Nitrogruppe oder eine Trifluormethylgruppe bedeutet,

R2 eine der Bedeutungen von R1 hat,
R3 ein Wasserstoffatom oder eine 1-4C-Alkylgruppe,
Y die Gruppierung -O-$(CH_2)_m$-,
m 0 oder eine ganze Zahl von 1 bis 4 und
n eine ganze Zahl von 2 bis 8 bedeuten,
wobei die Summe von m und n eine ganze Zahl von 2 bis 8 ist, und den pharmakologisch verträglichen Salzen der Carbonsäuren, zur Herstellung von Arzneimitteln für die Verhütung und/oder Behandlung vonkoronaren Herzkrankheiten.

7. Verwendung von Oxirancarbonsäuren der Formel I, worin R1 und R2 metaoder para-ständig sind und R1 ein Wasserstoffatom, ein Chloratom, eine Methylgruppe, eine Methoxygruppe, eine Nitrogruppe oder eine Trifluormethylgruppe, R2 ein Wasserstoffatom oder ein Chloratom, R3 ein Wasserstoffatom oder eine 1-4C-Alkylgruppe, Y die Gruppierung -O-$(CH_2)_m$-, m 0 oder 1 und n eine ganze Zahl von 3 bis 7 bedeuten, wobei die Summe von m und n eine ganze Zahl von 3 bis 7 ist, und den pharmakologisch verträglichen Salzen der Carbonsäuren, zur herstellung von Arzneimitteln für die Verhütung und/oder Behandlung von koronaren Herzkrankheiten.

8. Verwendung von Oxirancarbonsäuren der Formel I, worin R1 meta- oder para-ständig ist und R1 ein Wasserstoffatom, ein Chloratom oder eine Trifluormethylgruppe, R2 ein Wasserstoffatom, R3 ein Wasserstoffatom, eine Methylgruppe oder eine Ethylgruppe, Y die Gruppierung -O-, n eine ganze Zahl von 4 bis 6 bedeuten, und den pharmakologisch verträglichen Salzen der Carbonsäuren, zur Herstellung von Arzneimitteln für die Verhütung und/oder Behandlung von koronaren Herzkrankheiten.

9. Verwendung einer oder mehrerer Oxirancarbonsäuren ausgewählt aus der Gruppe bestehend aus 2-[4-(3-Chlorphenoxy)-butyl]-oxiran-2-carbonsäureethylester, 2-[4-(3-Trifluormethylphenoxy)-butyl]-oxiran-2-carbonsäureethylester, 2-[6-(4-Chlorphenoxy)-hexyl]-oxiran-2-carbonsäureethylester, 2-[5-(4-Chlorphenoxy)-pentyl]-oxiran-2-carbonsäureethylester, 2-[6-(3,4-Dichlorphenoxy)-hexyl]-oxiran-2-carbonsäureethylester, 2-[6-(4-Fluorphenoxy)-hexyl]-oxiran-2-carbonsäureethylester und 2-(6-Phenoxyhexyl)-oxiran-2-carbonsäureethylester sowie den entsprechenden Oxiran-2-carbonsäuren und ihren pharmakologisch verträglichen Salzen, zur Herstellung von Arzneimitteln für die Verhütung und/oder Behandlung von koronaren Herzkrankheiten.

10. Verwendung von 2-[6-(4-Chlorphenoxy)hexyl]-oxiran-2-carbonsäureethylester zur Herstellung von Arzneimitteln für die Verhütung und/oder Behandlung von koronaren Herzkrankheiten.

**Claims**

1. Use of oxirancarboxylic acids of Formula I

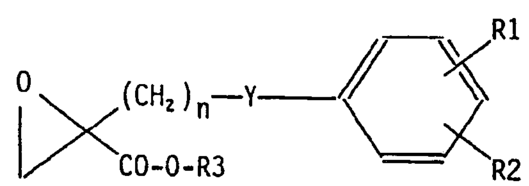

(I)

wherein
R1 is a hydrogen atom, a halogen atom, a 1-4C-alkyl group, a 1-4C-alkoxy group, a nitro group, or a trifluoromethyl group,
R2 has one of the meanings of R1,
R3 is a hydrogen atom or a 1-4C-alkyl group,
Y is the grouping -O-$(CH_2)_m$-,
m is 0 or a whole number from 1 to 4 and
n is a whole number from 2 to 8,
the sum of m and n being a whole number from 2 to 8, and the parmacologically tolerable salts of the carboxylic acids for the production of medicaments for the prevention and/or treatment of diseases based on a increased cholesterol and/or triglyceride concentration.

2. Use of oxirancarboxylic acids of Formula I, wherein R1 and R2 are in the meta- or para-position and R1 is a hydrogen atom, a chlorine atom, a methyl group, a methoxy group, a nitro group, or a trifluoromethyl group, R2 is a hydrogen atom or a chlorine atom, R3 is a hydrogen atom or a 1-4C-alkyl group, Y is the grouping -O-$(CH_2)_m$-, m is 0 or 1 and n is a whole number from 3 to 7, the sum of m and n being a whole number from 3 to

7, and the parmacologically tolerable salts of the carboxylic acids for the production of medicaments for the prevention and/or treatment of diseases based on an increased cholesterol and/or triglyceride concentration.

3. Use of oxirancarboxylic acids of Formula I, wherein R1 is in the meta- or para-position and R1 is a hydrogen atom, a chlorine atom, or a trifluoromethyl group, R2 is a hydrogen atom, R3 is a hydrogen atom, a methyl group, or an ethyl group, Y is the grouping -O-, n is a whole number from 4 to 6, and the parmacologically tolerable salts of the carboxylic acids for the production of medicaments for the prevention and/or treatment of diseases based on an increased cholesterol and/or triglyceride concentration.

4. Use of one or more oxirancarboxylic acids selected from the group consisting of ethyl 2-[4-(3-chlorophenoxy)-butyl]-oxiran-2-carboxylate, ethyl 2-[4-(3-trifluoromethylphenoxy)-butyl]-oxiran-2-carboxylate, ethyl 2-[6-(4-chlorophenoxy)-hexyl]-oxiran-2-carboxylate, ethyl 2-[5-(4-chlorophenoxy)-pentyl]-oxiran-2-carboxylate, ethyl 2-[6-(3,4-dichlorophenoxy)-hexyl]-oxiran-2-carboxylate, ethyl 2-[6-(4-fluorophenoxy)-hexyl]-oxiran-2-carboxylate and ethyl 2-(6-phenoxyhexyl)-oxiran-2-carboxylate as well as the corresponding oxiran-2-carboxylic acids and their pharmacologically tolerable salts, for the production of medicaments for the prevention and/or treatment of diseases based on an increased cholesterol and/or triglyceride concentration.

5. Use of ethyl 2-[6-(4-chlorophenoxy)-hexyl]-oxiran-2-carboxylate for the production of medicaments for the prevention and/or treatment of diseases based on an increased cholesterol and/or triglyceride concentration.

6. Use of oxirancarboxylic acids of Formula I,

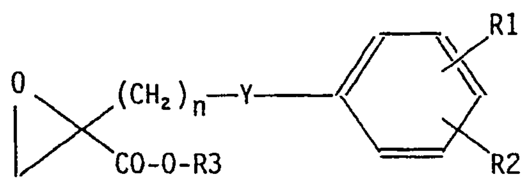

(I)

wherein

R1 is a hydrogen atom, a halogen atom, a 1-4C-alkyl group, a 1-4C-alkoxy group, a nitro group, or a trifluoromethyl group,

R2 has one of the meanings of R1,

R3 is a hydrogen atom or a 1-4C-alkyl group,

Y is the grouping -O- $(CH_2)_m$-,

m is 0 or a whole number from 1 to 4 and

n is a whole number from 2 to 8

the sum of m and n being a whole number from 2 to 8, and the parmacologically tolerable salts of the carboxylic acids for the production of medicaments for the prevention and/or treatment of coronary heart diseases.

7. Use of oxirancarboxylic acids of Formula I, wherein R1 and R2 are in the meta- or para-position and R1 is a hydrogen atom, a chlorine atom, a methyl group, a methoxy group, a nitro group, or a trifluoromethyl group, R2 is a hydrogen atom or a chlorine atom, R3 is a hydrogen atom or a 1-4C-alkyl group, Y is the grouping -O-$(CH_2)_m$-, m is 0 or 1 and n is a whole number from 3 to 7, the sum of m and n being a whole number from 3 to 7, and the pharmacologically tolerable salts of the carboxylic acids for the production of medicaments for the prevention and/or treatment of coronary heart diseases.

8. Use of oxirancarboxylic acids of Formula I, wherein R1 is in the meta- or para-position and R1 is a hydrogen atom, a chlorine atom, or a trifluoromethyl group, R2 is a hydrogen atom, R3 is a hydrogen atom, a methyl group, or an ethyl group, Y is the grouping -O-, n is a whole number from 4 to 6, and the parmacologically tolerable salts of the caboxylic acids for the production of medicaments for the prevention and/or treatment of coronary heart diseases.

9. Use of one or more oxirancarboxylic acids selected from the group consisting of ethyl 2-[4-(3-chlorophenoxy)-butyl]-oxiran-2-carboxylate, ethyl 2-[4-(3-trifluoromethylphenoxy)-butyl]-oxiran-2-carboxylate, ethyl 2-[6-(4-chlorophenoxy)-hexyl]-oxiran-2-carboxylate, ethyl 2-[5-(4-chlorophenoxy)-pentyl]-oxiran-2-carboxylate, ethyl 2-[6-(3,4-dichlorophenoxy)-hexyl]-oxiran-2-carboxylate, ethyl 2-[6-(4-fluorophenoxy)-hexyl]-oxiran-2-carboxylate and ethyl 2-(6-phenoxyhexyl)-oxiran-2-carboxylate as well as the corresponding oxiran-2-carboxylic acids and their pharmacologically tolerable salts for the production of medicaments for the prevention and/or treatment of coronary heart diseases.

10. Use of ethyl 2-[6-(4-chlorophenoxy)hexyl]-oxiran-2-carboxylate for the production of medicaments for the prevention and/or treatment of coronary heart diseases.

## Revendications

1. Utilisation d'acides oxirannecarboxyliques de formule I :

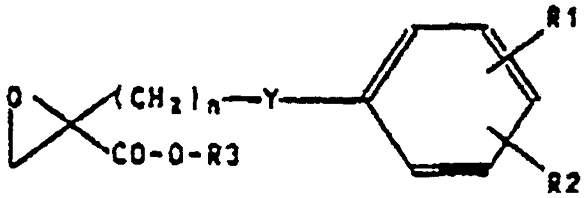

(I)

dans laquelle

R1 représente un atome d'hydrogène, un atome d'halogène, un groupe alkyle ayant de 1 à 4 atomes de carbone, un groupe alcoxy ayant de 1 à 4 atomes de carbone, un groupe nitro ou un groupe trifluorométhyle,

R2 a une des significations de R1,

R3 représente un atome d'hydrogène ou un groupe alkyle ayant de 1 à 4 atomes de carbone,

Y représente le groupe $-O-(CH_2)_{m-}$,

m vaut 0 ou représente un nombre entier valant de 1 à 4, et

n représente un nombre entier valant de 2 à 8, la somme de m et n étant un nombre entier valant de 2 à 8, et des sels pharmacologiquement acceptables des acides oxirannecarboxyliques, pour la préparation de médicaments destinés à la prévention et/ou au traitement de maladies qui reposent sur une concentration accrue du cholestérol et/ou des triglycérides.

2. Utilisation d'acides oxirannecarboxyliques de formule I, dans laquelle R1 et R2 sont en position méta ou para et R1 représente un atome d'hydrogène, un atome de chlore, un groupe méthyle, un groupe méthoxy, un groupe nitro ou un groupe trifluorométhyle, R2 représente un atome d'hydrogène ou un atome de chlore, R3 représente un atome d'hydrogène ou un groupe alkyle ayant de 1 à 4 atomes de carbone, Y représente le groupe $-O-(CH_2)_{m-}$, m vaut 0 ou 1, et n représente un nombre entier valant de 3 à 7, la somme de m et n étant un nombre entier valant de 3 à 7, et des sels pharmacologiquement acceptables des acides oxirannecarboxyliques, pour la préparation de médicaments destinés à la prévention et/ou au traitement de maladies qui reposent sur une concentration accrue du cholestérol et/ou des triglycérides.

3. Utilisation d'acides oxirannecarboxyliques de formule I, dans laquelle R1 est en position méta ou para, et R1 représente un atome d'hydrogène, un atome de chlore ou un groupe trifluorométhyle, R2 représente un atome d'hydrogène, R3 représente un atome d'hydrogène, un groupe méthyle ou un groupe éthyle, Y représente le groupe -O-, n représente un nombre entier valant de 4 à 6, et des sels pharmacologiquement acceptables des acides oxirannecarboxyliques, pour la préparation de médicaments destinés à la prévention et/ou au traitement de maladies qui reposent sur une concentration accrue du cholestérol et/ou des triglycérides.

4. Utilisation d'un ou plusieurs acide(s) oxirannecarboxylique(s) choisi(s) dans le groupe formé du 2-[4-(3-chlorophénoxy)butyl]-oxiranne-2-carboxylate d'éthyle, du 2-[4-(3-trifluorométhylphénoxy)butyl]-oxiranne-2-carboxylate d'éthyle, du 2-[6-(4-chlorophénoxy)hexyl]-oxiranne-2-carboxylate d'éthyle, du 2-[5-(4-chlorophénoxy)pentyl]-oxiranne-2-carboxylate d'éthyle, du 2-[6-(3,4-dichlorophénoxy)-hexyl]-oxiranne-2-carboxylate d'éthyle, du 2-[5-(4-fluorophénoxy)hexyl]-oxiranne-2-carboxylate d'éthyle et du 2-(6-phénoxyhexyl)-oxiranne-2-carboxylate d'éthyle, ainsi que des acides oxiranne-2-carboxyliques correspondants et de leurs sels phannacologiquement acceptables, pour la préparation de médicaments destinés à la prévention et au traitement de maladies qui reposent sur une concentration accrue du cholestérol et/ou des triglycérides.

5. Utilisation du 2-[6-(4-chlorophénoxy)hexyl]-oxiranne-2-carboxylate d'éthyle pour la préparation de médicaments destinés à la prévention et/ou au traitement de maladies qui reposent sur une concentration accrue du cholestérol et ou des triglycérides.

6. Utilisation d'acides oxirannecarboxyliques de formule I :

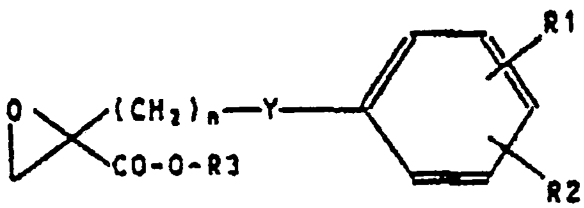

(I)

dans laquelle

R1 représente un atome d'hydrogène, un atome d'halogène, un groupe alkyle ayant de 1 à 4 atomes de carbone, un groupe alcoxy ayant de 1 à 4 atomes de carbone, un groupe nitro ou un groupe trifluorométhyle,

R2 a une des significations de R1,

R3 représente un atome d'hydrogène ou un groupe alkyle ayant de 1 à 4 atomes de carbone,

Y représente le groupe $-O-(CH_2)_{m}-$,

m vaut 0 ou représente un nombre entier valant de 1 à 4, et

n représente un nombre entier valant de 2 à 8, la somme de m et n étant un nombre entier valant de 2 à 8, et des sels pharmacologiquement acceptables des acides carboxyliques, pour la préparation de médicaments destinés à la prévention et/ou au traitement de maladies coronariennes.

7. Utilisation d'acides oxirannecarboxyliques de formule I, dans laquelle R1 et R2 sont en position méta ou para, et R1 représente un atome d'hydrogène, un atome de chlore, un groupe méthyle, un groupe méthoxy, un groupe nitro ou un groupe trifluorométhyle, R2 représente un atome d'hydrogène ou un atome de chlore, R3 représente un atome d'hydrogène ou un groupe alkyle ayant de 1 à 4 atomes de carbone, Y représente le groupe $-O-(CH_2)_{m}-$, m vaut 0 ou 1 et n représente un nombre entier valant de 3 à 7, la somme de m et n étant un nombre entier valant de 3 à 7, et des sels pharmacologiquement acceptables des acides carboxyliques, pour la préparation de médicaments destinés à la prévention et/ou au traitement de maladies coronariennes.

8. Utilisation d'acides oxirannecarboxyliques de formule I, dans laquelle R1 est en position méta ou para, et R1 représente un atome d'hydrogène, un atome de chlore ou un groupe trifluorométhyle, R2 représente un atome d'hydrogène, R3 représente un atome d'hydrogène, un groupe méthyle ou un groupe éthyle, Y représente le groupe -O-, n représente un nombre entier valant de 4 à 6, et des sels pharmacologiquement acceptables des acides carboxyliques, pour la préparation de médicaments destinés à la prévention et/ou au traitement de maladies coronariennes.

9. Utilisation d'un ou plusieurs acide(s) oxirannecarboxylique(s) choisi(s) dans le groupe formé du 2-[4-(3-chlorophénoxy)butyl]-oxiranne-2-carboxylate d'éthyle, du 2-[4-(3-trifluorométhylphénoxy)butyl]-oxiranne-2-carboxylate d'éthyle, du 2-[6-(4-chlorophénoxy)hexyl]-oxiranne-2-carboxylate d'éthyle, du 2-[5-(4-chlorophénoxy)pentyl]-oxiranne-2-carboxylate d'éthyle, du 2-[6-(3,4-dichlorophénoxy)-hexyl]-oxiranne-2-carboxylate d'éthyle, du 2-[5-(4-fluorophénoxy)hexyl]-oxiranne-2-carboxylate d'éthyle et du 2-(6-phénoxyhexyl)-oxiranne-2-carboxylate d'éthyle, ainsi que des acides oxiranne-2-carboxyliques correspondants et de leurs sels pharmacologiquement acceptables, pour la préparation de médicaments destinés à la prévention et/ou au traitement de maladies coronariennes.

10. Utilisation du 2-[6-(4-chlorophénoxy)hexyl]-oxiranne-2-carboxylate d'éthyle pour la préparation de médicaments destinés à la prévention et/ou au traitement de maladies coronariennes.